# FASCICULE DE BREVET EUROPEEN

(11) **EP 1 485 719 B1**
(45) Date de publication et mention de la délivrance du brevet: **27.02.2008**
(21) Numéro de dépôt: 03732631.1
(22) Date de dépôt: 27.03.2003
(51) Int. Cl.: G01N 33/68, C07K 7/06, G01N 33/574, C07K 14/82, C07K 14/47

(54) **EPITOPES PEPTIDIQUES COMMUNS A DES ANTIGENES D'UNE MEME FAMILLE MULTIGENIQUE**
GEMEINSAME EPITOPE VON ANTIGENEN AUS EINER MULTIGEN-FAMILIE
PEPTIDE EPITOPES COMMON TO ANTIGENS OF THE SAME MULTIGENE FAMILY

(30) Priorité: 28.03.2002 FR 0203888
(43) Date de publication de la demande: 15.12.2004
(73) Titulaire: Institut Gustave Roussy, 94805 Villejuif Cédex (FR); INSTITUT NATIONAL DE LA SANTE ET DE LA RECHERCHE MEDICALE (INSERM), 75013 Paris (FR)
(72) Inventeur: KOSMATOPOULOS, Kostas, F-75013 Paris (FR); GRAFF-DUBOIS, Stéphanie, F-75013 Paris (FR)
(74) Mandataire: Vialle-Presles, Marie José
(86) Numéro de dépôt international: PCT/FR2003/000970
(87) Numéro de publication internationale: WO 2003/083124

(56) Documents cités:
- WO-A-00/20581
- WO-A-00/52045
- WO-A-01/41787
- WO-A-01/42267
- TANZARELLA SILVIA ET AL: "Identification of a promiscuous T-cell epitope encoded by multiple members of the MAGE family." CANCER RESEARCH, vol. 59, no. 11, 1 juin 1999 (1999-06-01), pages 2668-2674, XP001132068 ISSN: 0008-5472 cité dans la demande
- GRAFF-DUBOIS, STEPHANIE ET AL: "Generation of CTL recognizing an HLA-A*0201-restricted epitope shared by MAGE-A1, -A2, -A3, -A4, -A6, -A10, and -A12 tumor antigens: implication in a broad-spectrum tumor immunotherapy" JOURNAL OF IMMUNOLOGY, 169(1), 575-580, 1 juillet 2002 (2002-07-01), XP001109368

## Description

La présente invention est relative à des peptides représentant des épitopes partagés d'antigènes tumoraux, et à leur utilisation en immunothérapie.

La vaccination ou immunothérapie peptidique est une approche thérapeutique qui fait actuellement l'objet d'un grand intérêt dans le cadre de la prévention ou du traitement des cancers. Son principe repose sur l'immunisation par des peptides reproduisant des épitopes T d'antigènes tumoraux reconnus par les lymphocytes T cytotoxiques (CTL), qui jouent un rôle majeur dans l'élimination des cellules cancéreuses exprimant ces antigènes à leur surface.

On rappellera que les CTL ne reconnaissent pas les antigènes protéiques entiers, mais des fragments peptidiques de ceux-ci, présentés par les molécules du complexe majeur d'histocompatibilité (CMH) exprimées à la surface de différentes cellules. Ce sont ces fragments peptidiques qui constituent les épitopes T. Les peptides présentés par le complexe majeur d'histocompatibilité de classe I (CMH I) ont généralement 8 à 11 acides aminés, et sont reconnus par les cellules T CD8+, qui représentent la composante majeure de la réponse cytotoxique. Les peptides présentés par le complexe majeur d'histocompatibilité de classe II (CMH II) ont généralement 13 à 18 acides aminés et sont reconnus par les cellules T CD4+.

L'identification de ces épitopes constitue donc une étape essentielle pour le développement de compositions d'immunothérapie anti-tumorale. Compte tenu du rôle essentiel de la réponse CD8+ dans la cytotoxicité, l'identification d'épitopes présentés par le CMH I est essentielle. Le rôle des lymphocytes T CD4+ dans l'immunité anti-tumorale n'est toutefois pas négligeable. Aussi, plusieurs équipes ont recherché des épitopes d'antigènes tumoraux présentés par le CMH II. Les documents WO00/52045 et WO 00/20581 décrivent des peptides dérivés de l'antigène MAGE-3, et présentés par le CMH II.

De nombreux antigènes tumoraux sont connus à l'heure actuelle ; certains des épitopes T de ces antigènes ont été identifiés, et l'efficacité de vaccins à base de peptides reproduisant ces épitopes T a été montrée dans de nombreux cas.

Cependant, l'expression de la majorité des antigènes tumoraux est restreinte à certains types histologiques de tumeurs, ce qui limite leur utilisation clinique.

Une difficulté supplémentaire provient du formidable polymorphisme des molécules du CMH, car l'efficacité de liaison et de présentation d'un peptide donné n'est pas la même pour différente molécules du CMH. Le document WO 01/42267 décrit des épitopes identifiés et, le cas échéant, optimisés pour se lier à plusieurs molécules du CMH.

Une autre limitation importante de l'immunothérapie peptidique résulte de l'apparition chez certains patients, de variants de la tumeur (variants d'échappement) qui n'expriment plus l'antigène reconnu par les lymphocytes T cytotoxiques.

Certains antigènes tumoraux appartiennent à des familles multigéniques : à l'intérieur d'une même famille existe une homologie de séquence, qui peut se traduire par l'existence d'épitopes partagés, communs à deux ou plusieurs membres d'une même famille.

Généralement, différents membres d'une même famille d'antigènes sont exprimés dans différents types de tumeurs ; l'utilisation d'un épitope partagé par ces antigènes pourrait permettre d'obtenir des vaccins anti-tumoraux à large spectre d'activité.

Par ailleurs, dans de nombreux cas, plusieurs antigènes de la même famille sont co-exprimés dans une même lignée tumorale ; la probabilité de perte de l'expression de l'ensemble de ces antigènes étant extrêmement faible, l'utilisation d'un épitope partagé par ces antigènes peut permettre d'éviter l'apparition de variants d'échappement.

Parmi les antigènes tumoraux connus pour appartenir à une famille multigénique, on citera notamment les antigènes des familles MAGE-A, HER, BAGE, ou GAGE.

MAGE-A est une famille multigénique constituée de 12 gènes homologues (MAGE-A1 à A12) localisés dans la région q28 du chromosome X (DE PLAEN et al., Immunogenetics. 40, 360, 1994). Parmi les membres de cette famille, MAGE-A1,-A2, - A3, - A4, - A6, - A10 et - A12 sont exprimés fortement par les tumeurs mais pas par les tissus normaux, à l'exception des testicules et du placenta.

Les antigènes MAGE-A 1, 2, 3, 4, 6 et 12 sont présents dans un large spectre de tumeurs d'origine histologique très variée comme les mélanomes, les cancers du poumon, les cancers du sein, les tumeurs tête et cou, et les sarcomes, les myélomes, etc... (BRASSEUR et al., Int. J Cancer, 52, 839, 1992 ; BRASSEUR et al., Int. J. Cancer, 63, 375, 1995 ; PELLAT-DECEUNYNCK et al., Eur. J. Immunol., 30, 803, 2000 ; OFUJI et al., Anticancer Res., 18, 3639, 1998 ; GIBBS et al., Melanoma Res., 10, 259, 2000 ; PATARD et al., Int. J. Cancer, 64, 60, 1995 ; OTTE et al., Cancer Res., 61, 6682, 2001 ; SUDO et al., J. Orthop. Res., 15, 128, 1997 ; LEE, et al., Acta. Otolaryngol., 116, 633, 1996 ; HASEGAWA et al., Arch. Pathol. Lab. Med., 122, 551, 1998 ; YAMANAKA et al., Int. J. Mol. Med., 2, 57, 1998 ; GILLESPIE et al., Br. J. Cancer., 78, 816, 1998 ; TAHARA et al., Cancer, 85, 1234, 1999).

L'expression de chaque antigène MAGE-A peut varier d'une tumeur à l'autre, mais globalement, la grande majorité des tumeurs exprime au moins un antigène MAGE-A.

Les protéines de la famille HER sont également exprimées par un grand nombre de tumeurs d'origine variée telles que la tumeur du sein, de l'ovaire, de la vessie, du colon, etc. (J. Cancer Res. Clin. Oncol. 2000, 126, 205 ; Clin. Cancer Res. 1999, 5, 4164 ; Clin. Cancer Res. 1999, 5, 3653 ; J. Cell Biochem. 1999, 73, 522 ; Br. J. Cancer 1994, 70, 459 ; Cancer Res. 2000, 60, 1483 ; Clin. Cancer Res. 2001, 7, 1957 ; Adv. Cancer Res. 1997, 71, 343 ; Stem cells, 1997, 15, 1 ; Science 1987, 235, 177 ; Cancer Res. 1990, 50, 4087).

Malgré l'intérêt potentiel de l'utilisation d'épitopes T partagés, cette approche n'a été que très rarement proposée. On citera les résultats de TANZARELLA et al. (Cancer Res., 59, 2668-74, 1999), qui décrivent un peptide de séquence REPVTKAEML (SEQ ID No : 38), constituant un épitope commun à plusieurs antigènes de la famille MAGE-A (MAGE-A 1, 2, 3 et 6) et se liant à l'allèle HLA-B*3701 du CMH-I.

Le premier obstacle à la mise en évidence d'autres peptides constituant des épitopes communs à plusieurs antigènes tumoraux d'une même famille, est la rareté des régions de taille appropriée (au moins 8 acides aminés pour un peptide présenté par le CMH I) totalement identiques d'un antigène à l'autre.

Pour surmonter cet obstacle, les Inventeurs ont eu l'idée de rechercher si des peptides présentant un pourcentage plus faible d'identité pouvaient posséder une spécificité antigénique commune, et ont trouvé qu'il suffisait d'une identité limitée à la séquence de 5 acides aminés s'étendant des positions P4 à P8 du peptide.

On définit ici comme « séquence s'étendant des positions P4 à P8 » la séquence commençant par l'acide aminé situé en position 4 à compter de l'extrémité N-terminale du peptide, et se terminant par l'acide aminé situé en position 8 à compter de l'extrémité N-terminale du peptide.

La présente description divulgue un procédé d'identification d'épitopes peptidiques présentés par une molécule HLA de classe I et partagés par au moins deux antigènes d'une même famille multigénique, caractérisé en ce qu'il comprend au moins les étapes suivantes :
a) l'alignement des séquences desdits antigènes pour identifier sur chacun d'entre eux une séquence de 8 à 10 acides aminés possédant les caractéristiques suivantes :
   - elles comprennent au moins une séquence pentapeptidique commune précédée par 3 acides aminés à l'extrémité N-terminale, et éventuellement suivie par 1 ou 2 acides aminés à l'extrémité C-terminale ;
   - au moins deux desdites séquences diffèrent entre elles par au moins un acide aminé positionné en dehors de ladite séquence pentapeptidique ;
b) la préparation des peptides répondant aux séquences identifiées à l'étape a).

Selon un mode de mise en oeuvre préféré du procédé conforme à l'invention, il comprend en outre une étape c) de détermination de l'affinité de liaison de chacun des peptides préparés à l'étape b) pour la molécule HLA de classe I concernée, et de la stabilité du complexe peptide/molécule HLA de classe I.

Cette étape permet l'évaluation de l'immunogénicité potentielle des peptides.

En effet, des peptides non-immunogènes présentent le plus souvent une faible affinité pour la molécule HLA de classe I, et/ou forment avec celle-ci un complexe peu stable. Des méthodes pour déterminer l'affinité du peptide pour la molécule HLA, et la stabilité du complexe formé sont connues en elles-mêmes. On citera par exemple celle décrite par FIRAT et al. (Eur. J. Immunol., 29, 3112, 1999).

L'affinité d'un peptide pour une molécule HLA est le plus souvent définie par rapport à celle d'un peptide de référence par exemple IVGAETFYV (SEQ ID No : 1) pour HLA-A*0201 ou RPHERNGFTV (SEQ ID No : 2) pour HLA-B*0702), sous forme d'affinité relative. L'affinité relative est définie comme le rapport entre la concentration du peptide testé et la concentration du peptide de référence permettant la formation dans les mêmes conditions, de la même quantité de complexe peptide/molécule HLA de classe I. Plus l'affinité relative est importante, plus l'affinité de liaison du peptide pour la molécule HLA de classe I sera faible.

La stabilité du complexe peptide/molécule HLA de classe I. est souvent définie par la DC50, qui représente le temps nécessaire à la dissociation de 50% des complexes formés.

Généralement, l'affinité relative est inférieure à 5 et la DC₅₀ supérieure à 2 heures dans le cas des peptides potentiellement immunogènes.

Si la mise en oeuvre de l'étape c) fait apparaître un ou plusieurs peptides potentiellement immunogène(s), l'immunogénicité de ceux-ci peut être vérifiée, par exemple par des méthodes classiques de détermination de la capacité de ce peptide à générer, *in vivo, ex vivo*, ou *in vitro* une réponse CTL spécifique vis-à-vis de cellules-cibles chargées avec ce peptide, ou exprimant l'antigène dont il est issu, ou d'autres antigènes de la même famille.

Si aucun peptide potentiellement immunogène n'est mis en évidence à l'étape c) le procédé conforme à l'invention comprend une étape supplémentaire de préparation d'un peptide variant à partir des peptides obtenus à l'étape b) par substitution d'un ou plusieurs des acides aminés situés en dehors de la séquence pentapeptidique commune, par un ou des acides aminés favorables à l'immunogénicité, par exemple, par un des résidus du motif d'ancrage défini pour la molécule HLA de classe I concernée. On peut également substituer l'acide aminé N-terminal par une tyrosine, comme décrit dans la Demande PCT WO 02/08716.

L'affinité de ce peptide variant pour la molécule concernée peut être déterminée comme indiqué ci-dessus. Son immunogénicité sera ensuite vérifiée en déterminant sa capacité à générer une réponse CTL spécifique vis-à-vis du peptide natif dont il est issu, ainsi que de l'antigène dont provient ce peptide natif, et des autres antigènes de la même famille.

La mise en oeuvre du procédé décrit dans la description a ainsi permis aux Inventeurs d'identifier des épitopes partagés par les antigènes de la famille MAGE-A, ainsi que des épitopes partagés par des antigènes de la famille HER.

Les Inventeurs ont en particulier obtenu, à partir d'une région commune aux antigènes MAGE-A1, -2, -3, -4, -6, -10 et -12 de la famille MAGE-A, un peptide immunogène présenté par HLA-A*0201, et capable d'induire des lymphocytes T cytotoxiques reconnaissant tous les antigènes MAGE-A, et de lyser des cellules tumorales exprimant au moins un antigène de la famille MAGE-A.

Ce peptide, qui est défini par la séquence (code 1 lettre) : YLEYRQVPV (SEQ ID No : 3), fait partie de l'objet de la présente invention.

De même, les Inventeurs ont obtenu, à partir d'une région commune aux antigènes HER1, 2, 3 et 4, un peptide immunogène présenté par HLA-A*0201, et capable d'induire des lymphocytes T cytotoxiques reconnaissant tous les antigènes HER1, 2, 3 ou 4 et de lyser des cellules exprimant au moins un antigène de la famille HER.

Ce peptide, qui est défini par la séquence (code 1 lettre) : YVWELMTFGV (SEQ ID No : 4), fait également partie de l'objet de la présente invention.

Les peptides YLEYRQVPV (SEQ ID No : 3) et YVWELMTFGV (SEQ ID No : 4), sont utilisables dans le cadre de l'immunothérapie anti-tumorale, pour induire une réponse CTL à large spectre, permettant le traitement d'une grande variété de tumeurs. En outre, dans le cas de peptides, comme le peptide YLEYRQVPV (SEQ ID No : 3), dérivés de la famille MAGE-A dont l'expression, en dehors des testicules et du placenta qui sont des tissus immunoprivilégiés, se limite aux seuls tissus tumoraux, le risque de réaction auto-immune est considérablement réduit.

La présente invention a également pour objet des compositions comprenant au moins un peptide immunogène conforme à l'invention.

Il peut s'agir de compositions multiépitopiques, capables de générer une réponse CTL polyspécifique, et qui dans ce but comprennent également un ou plusieurs autre(s) épitope (s) immunogène (s) . Ces épitopes peuvent être issus du même antigène, ou de deux ou plusieurs antigènes différents.

Ces compositions multiépitopiques conformes à l'invention peuvent également comprendre au moins un épitope présenté par une molécule du CMH II, et capable d'induire une réponse T auxiliaire. Elles peuvent comprendre en outre, pour être plus largement utilisables sur une population dont les individus portent des allèles HLA différents, un ou plusieurs épitopes présentés par des molécules du CMH I autres que HLA-A*0201.

Selon un mode de réalisation préféré d'une composition conforme à l'invention, elle comprend au moins un polypeptide chimérique comprenant une ou plusieurs copies d'un peptide immunogène conforme à l'invention. Dans le cas d'une composition multiépitopique, ledit polypeptide chimérique comprend en outre une ou plusieurs copies d'au moins un autre épitope immunogène.

Un tel polypeptide chimérique peut être facilement obtenu par des méthodes connues en elles-mêmes, et notamment par les techniques classiques de l'ADN recombinant.

La présente invention a également pour objet les molécules d'acide nucléique codant pour un peptide immunogène ou pour un polypeptide chimérique conforme à l'invention.

La présente invention a également pour objet l'utilisation d'un épitope peptidique immunogène, d'une composition, ou d'une molécule d'acide nucléique conforme à l'invention pour l'obtention d'un médicament, et notamment d'un médicament destiné à l'immunothérapie antitumorale.

La présente invention englobe également les médicaments comprenant, en tant que principe actif, au moins un peptide immunogène, une composition, ou une molécule d'acide nucléique conforme à l'invention.

Des médicaments conformes à l'invention peuvent comprendre en outre les excipients usuels, ainsi que des adjuvants habituellement utilisés en immunothérapie, et permettant par exemple de favoriser l'administration du principe actif, de le stabiliser, d'augmenter son immunogénicité, etc.

La présente invention sera mieux comprise à l'aide du complément de description qui va suivre, qui se réfère à des exemples non-limitatifs de mise en oeuvre du procédé conforme à l'invention pour identifier des épitopes partagés dans la famille MAGE-A et dans la famille HER.

### EXEMPLE 1 : IDENTIFICATION D'EPITOPES PRESENTES PAR LA MOLECULE HLA-A*0201 PARTAGES PAR LES ANTIGENES MAGE-A 1, 2, 3, 4, 6, 10 ET 12.

### I : Identification de peptides candidats :

Pour identifier un ou plusieurs épitopes partagés par les différents antigènes MAGE-A et présentés par la molécule HLA-A*0201, les séquences des antigènes MAGE-A ont été alignées, et des régions de 9 à 10 acides aminés ont été sélectionnées sur la base de leur homologie entre ces antigènes.

Dans le descriptif qui suit, ces régions de 9 à 10 acides aminés sont désignées par référence à la position de leur premier acide aminé dans la séquence MAGE-A1.

Un seul peptide, p262, a été retrouvé à l'identique dans toutes les séquences MAGE-A. Un autre peptide, p174, a été retrouvé à l'identique dans les séquences MAGE-A1, -A2,-A3,-A4,-A6,-A12

Pour élargir le choix des peptides candidats, une seconde recherche a été effectuée pour sélectionner des régions présentant une complète identité de séquences entre les positions P4 et P8. Deux régions respectaient ce critère : 248 et 264. Les séquences des groupes de peptides issus de ces régions, ainsi que celle des peptides p174 et p262 sont représentées sur le Tableau I.

**Tableau I**

| Peptides | Séquences | Antigène MAGE-A |
|---|---|---|
| 174 | CLGLSYDGLL (SEQ ID No : 5) | A1, A2, A3, A4, A6, A12 |
| 248 | YLEYRQVPG (SEQ ID No: 6) | A2, A3, A4, A6, A10, A12 |
| | YLEYRQVPD (SEQ ID No : 7) | A1 |
| 262 | YEFLWGPRA (SEQ ID No : 8) | A1, A2, A3, A4, A6, A10, A12 |
| 264 | FLWGPRALV (SEQ ID No : 9) | A3, A12 |
| | FLWGPRALI (SEQ ID No : 10) | A2, A6 |
| | FLWGPRALA (SEQ ID No : 11) | A1, A4 |

Le groupe p248 est composé de deux peptides : p248G9 (MAGE-A2,-A3,-A4,-A6,-A10,-A12) et p248D9 (MAGE-A1) différant par leur résidu C-terminal (position P9). Le groupe p264 a été exclu de la suite de l'étude, car il correspond à un épitope déjà connu de MAGE-A3 (FLWGPRALV (SEQ ID No : 9)) restreint à HLA-A*0201, et il est également connu qu'il n'est pas apprêté efficacement par les cellules tumorales (MICONNET *et al.,* J. Biol. Chem., 275, 26892, 2000).

### II : Affinité pour HLA-A*0201 des peptides sélectionnés, et construction de variants potentiellement plus immunogènes.

L'affinité des peptides sélectionnés pour HLA-A*0201 a été définie par deux paramètres : l'affinité relative (RA) qui reflète la capacité des peptides à se fixer à HLA-A*0201, et la vitesse de dissociation des complexes HLA-A*0201/peptide (DC₅₀) qui témoigne de leur stabilité. Les peptides à affinité élevée (RA<5 et DC₅₀>2 hrs), sont potentiellement immunogènes, contrairement aux peptides à faible affinité (RA>5 et DC₅₀<2 hrs).

### Affinité relative :

Des cellules T2 (FIRAT et al., Eur. J. Immunol., 29, 3112, 1999) (3x10⁵ cellules/ml) humaines, qui sont déficientes en transporteurs TAP, sont incubées à 37°C pendant 16 heures avec diverses concentrations (100 µM, 10 µM, 1 µM, 0,1 µM) de chaque peptide à tester dans du milieu RPMI 1640 sans sérum, supplémenté avec 100 ng/ml de β2-microglobuline humaine. Ensuite, elles sont lavées deux fois, et marquées avec l'anticorps monoclonal BB7.2 (PARHAM et al., Hum. Immunol., 3, 4, 277-299, 1981) qui est spécifique de la molécule HLA-A*0201, puis avec un anticorps de chèvre anti-Ig de souris, couplé à l'isothiocyanate de fluorescéine (FITC).

Les cellules sont ensuite analysées en cytométrie de flux. Pour chaque concentration de peptide, la fluorescence spécifique de HLA-A*0201 est calculée en tant que pourcentage de la fluorescence obtenue avec 100 µM d'un peptide de référence (HIVpol 589 ; IVGAETFYV (SEQ ID No : 1)). L'affinité relative (RA) est définie comme le rapport de la concentration de chaque peptide induisant 20% de la fluorescence obtenue avec 100 µM du peptide de référence, à la concentration du peptide de référence induisant 20% de la fluorescence obtenue avec 100 µM dudit peptide de référence. Plus l'affinité relative est faible, et plus fortement le peptide se lie à HLA-A*0201. La RA moyenne pour chaque peptide est déterminée à partir d'au moins trois expériences indépendantes. Dans toutes les expériences, 20% de la fluorescence maximale ont été obtenus pour 1 à 3 µM du peptide de référence.

### Stabilité :

Des cellules T2 (10⁶/ml) sont incubées pendant une nuit à 37°C avec 100 µM de chaque peptide à tester dans du milieu RPMI 1640 sans sérum, supplémenté avec 100 ng/ml de β2-microglobuline humaine. Ensuite, elles sont lavées à quatre reprises pour éliminer les peptides libres, incubées avec du BREFELDIN A (SIGMA ; 10 µg/ml) pendant une heure pour prévenir l'expression à leur surface des molécules HLA-A*0201 nouvellement synthétisées, lavées et incubées à 37°C pendant 0, 2, 4, 6 ou 8 heures. Pour chaque temps d'incubation, les cellules sont ensuite marquées, comme indiqué ci-dessus, avec l'anticorps BB7.2, et analysées en cytométrie de flux pour évaluer la quantité de complexe peptide/HLA-A*0201 présent à leur surface. Cette quantité est évaluée par la formule : (fluorescence moyenne des cellules T2 préincubées avec le peptide - fluorescence moyenne des cellules T2 traitées dans des conditions similaires en l'absence de peptide). Le DC₅₀ (complexe de dissociation : DC) est défini comme étant le temps requis pour la perte de 50% des complexes HLA-A*0201/peptide stabilisés à t=0.

Les résultats de ces expérimentations, présentés dans le Tableau II ci-après, montrent que les peptides p174, p262, p248G9 et p248D9 n'ont qu'une affinité faible pour HLA-A*0201.

Pour augmenter cette affinité, et par conséquent leur immunogénicité, les peptides p262, p248G9 et p248D9 ont été modifiés en remplaçant les résidus situés aux positions P2 ou C-terminale, par des résidus d'ancrage spécifiques de l'allèle HLA-A*0201, pour produire les variants p262L2 et p248V9. Pour le peptide p174, deux variants ont été produits : dans l'un (p174Y1), le résidu en position P1 a été remplacé par une tyrosine, dans le second (P174Y1V10), le résidu C-terminal P10 a en outre été remplacé par le résidu valine, susceptible de permettre un ancrage plus fort.

L'affinité de ces variants pour HLA-A*0201 a été déterminée comme décrit ci-dessus.

Les résultats, résumés dans le Tableau II, montrent que les variants p262L2 et p248V9 possèdent une affinité de liaison importante (RA = 0,2 et 1,8 respectivement) et forment des complexes stables (DC₅₀= 6h et 4h respectivement). P174Y1V10 possède également une affinité de liaison importante (RA= 2,5) mais la stabilité du complexe formé apparaît insuffisante.

**Tableau II**

| Peptides | Séquence | MAGE-A | RA | DC₅₀ |
|---|---|---|---|---|
| p174 | CLGLSYDGLL (SEG ID No : 5) | 1,2,3,4,6,12 | 41 | <2 |
| p174Y1 | YLGLSYDGLL (SEQ ID No : 12) | | 13 | <2 |
| p174Y1V10 | YLGLSYDGLV (SEQ ID No : 13) | | 2.5 | <2 |
| p248G9 | YLEYRCQVPG (SEQ ID No : 6) | 2,3,4,6,10,12 | >27 | <2 |
| p248D9 | YLEYRQVPD (SEQ ID No : 7) | 1 | 22.5 | <2 |
| p248V9 | YLEYRQVPV (SEQ ID No :3) | | 1.8 | 4 |
| p262 | YEFLWGPRA (SEQ ID No: 8) | 1,2,3,4,6,10,12 | >35 | <2 |
| p262L2 | YLFLWGPRA (SEQ ID No : 14) | | 0.2 | 6 |

### EXEMPLE 2 : IMMUNOGENICITE DES PEPTIDES MAGE-A VARIANTS :

### Induction de CTL spécifiques par vaccination avec les peptides variants

L'immunogénicité des peptides variants p174Y1V10, p262L2, et p248V9 a été évaluée par génération de CTL sur des souris transgéniques HHD (PASCOLO et al., J. Exp. Med., 185, 2043, 1997). Ces souris sont β2m-/-,D ^{b}-/- et expriment une monochaîne HLA-A*0201 composée des domaines α1 et α2 de HLA-A*0201 et des domaines α3 et intracellulaire de D^{b}, reliée par son N-terminal au C-terminal de la β2-microglobuline humaine par un peptide de 15 acides aminés.

Les souris HHD reçoivent une injection sous-cutanée à la base de la queue avec 100 µg de chaque peptide variant à tester émulsifié dans de l'adjuvant incomplet de Freund, en présence de 140 pg d'un épitope auxiliaire T dérivé de l'antigène « core » de HBV (128-140, séquence TPPAYRPPNAPIL).

Après 11 jours, des cellules spléniques prélevées sur les souris (5×10⁷ cellules dans 10 ml) sont stimulées *in vitro* avec le peptide à tester (10 µM). Au 6ème jour de culture, les populations qui répondent sont testées pour déterminer une cytotoxicité spécifique. Les cellules qui répondent sont restimulées *in vitro* à des intervalles d'une semaine avec 2×10⁷ cellules spléniques HHD irradiées (3000 rads) et 1 à 0,1 µM de peptide en présence de 50 UI/ml d'IL2 recombinante (PROLEUKIN, CHIRON CORP).

Des essais de cytotoxicité sont effectués 6 jours après la dernière stimulation.

Des cellules RMAS-HHD sont utilisées comme cibles pour étudier la cytotoxicité. Ces cellules sont obtenues par transfection de cellules RMAS murines avec la construction HHD comme décrit par PASCOLO et al. (J. Exp. Med., 185, 2043, 1997).

Ces cellules-cibles sont marquées avec 100 µCi de ⁵¹Cr pendant 90 minutes, puis lavées trois fois et étalées dans des plaques de 96 puits à fond rond (3×10³ cellules/puits dans 100 µl de RPMI 1640 + 3% de sérum de veau foetal). Elles sont chargées avec différentes concentrations de peptide à tester (variant, peptide natif, ou peptide témoin non-pertinent), à 37°C pendant 90 minutes.

Ensuite, 100 µl des cellules effectrices (rapport cellules effectrices/cellules cible = 40/1) sont ajoutés dans les puits et les plaques sont incubées à 37°C pendant 4 heures. Après incubation, 100 µl de surnageant sont collectés et la radioactivité est mesurée dans un compteur γ.

Le pourcentage de lyse spécifique est calculé par la formule : [(libération de ⁵¹Cr expérimentale-libération de ⁵¹Cr spontanée)/(libération de ⁵¹Cr maximale-libération de ⁵¹Cr spontanée)] × 100. Dans toutes les expériences, la libération spontanée est inférieure à 20% de la libération maximale induite par HCl 3N.

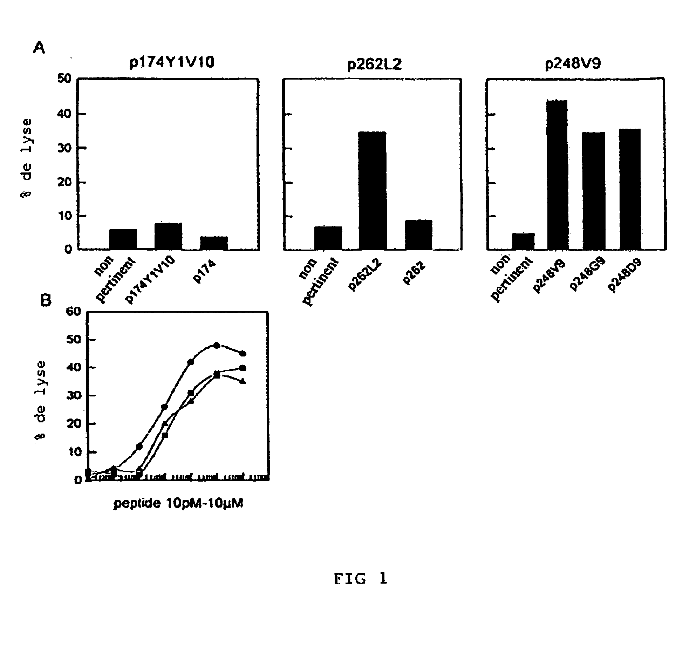

Les résultats de ces expérimentations sont illustrés par la Figure 1A.

Le peptide p174Y1V10 n'est pas capable de générer une réponse CTL, ce qui était prévisible au vu de la faible stabilité de son complexe avec HLA-A*0201.

Le peptide p262L2 induit des CTL capables de reconnaître ce peptide, mais pas le peptide natif p262.

En revanche, le peptide p248V9 induit une réponse CTL dirigée non seulement contre ce variant, mais également contre les deux peptides natifs p248G9 et p248D9.

Ces résultats montrent que le variant p248V9 génère des CTL qui tuent les cibles RMAS-HHD chargées avec le peptide variant, ou avec le peptide natif correspondant.

Dans le cas du peptide p262L2, la non-conservation de l'antigénicité du peptide natif peut s'expliquer par la modification de structure très importante résultant de la substitution en position P2 d'un résidu glutamate par un résidu leucine, de taille et de charge très différentes.

### Reconnaissance des épitopes apprêtés naturellement d'antigènes MAGE-A par des CTL induites par le peptide p248V9

Une lignée de CTL dénommée CTL248, a été établie à partir des cellules spléniques d'une souris HDD immunisée avec le peptide p248V9, par stimulation répétées *in vitro* avec des concentrations décroissantes de p248G9 (10 µM-1 µM). Cette lignée est maintenue en culture en présence de 1 µM du peptide p248G9.

Pour tester si les cellules de la lignée CTL248 pouvaient reconnaître des antigènes MAGE-A apprêtés naturellement, deux types d'expérimentations ont été effectués.

### 1) Stimulation par des cellules COS-7 transfectées exprimant des antigènes MAGE-A.

Les cellules de la lignée CTL248 sont stimulées, pour chacun des antigènes MAGE-A1,-A2,A3,-A4,-A6,-A10 et - A12, avec des cellules COS-7 de singe co-transfectées avec la construction HHD (PASCOLO *et al.* précité) et un plasmide contenant l'ADNc dudit antigène. A titre de témoins négatifs on utilise des cellules COS-7 transfectées soit avec la construction HHD seule, soit avec l'ADNc de l'antigène MAGE-A concerné. La stimulation des CTL est évaluée par mesure de leur sécrétion de TNF-α. A titre de témoin positif, on utilise les cellules COS-7 transfectées avec la construction HHD et chargées avec les peptides p248D9 et p248G9.

4 jours après la transfection, les cellules COS-7 sont mises en contact avec les cellules CTL248 à raison de 5×10⁴ CTL pour 3×10⁴ cellules COS-7 dans du RPMI 1640 en présence de 10% SVF.

Après 6 heures d'incubation, le surnageant est prélevé et mis en contact avec des cellules de fibrosarcome de souris WEHI164 clone 13 (3×10⁴ par puits) qui se caractérisent par une forte sensibilité à l'apoptose induite par le TNF-α. Afin de quantifier la teneur en TNF des surnageants de culture, une gamme étalon de TNF-α (concentrations de 0 à 10⁴ pg/mL) est utilisée en parallèle. Après 16 heures d'incubation à 37°C, la viabilité des cellules WEHI-164 clone 13 est déterminée par un test colorimétrique au MTT (SIGMA) (ESPEVIK et NISSEN MEYER, J. Immunol. Methods., 95, 99, 1986).

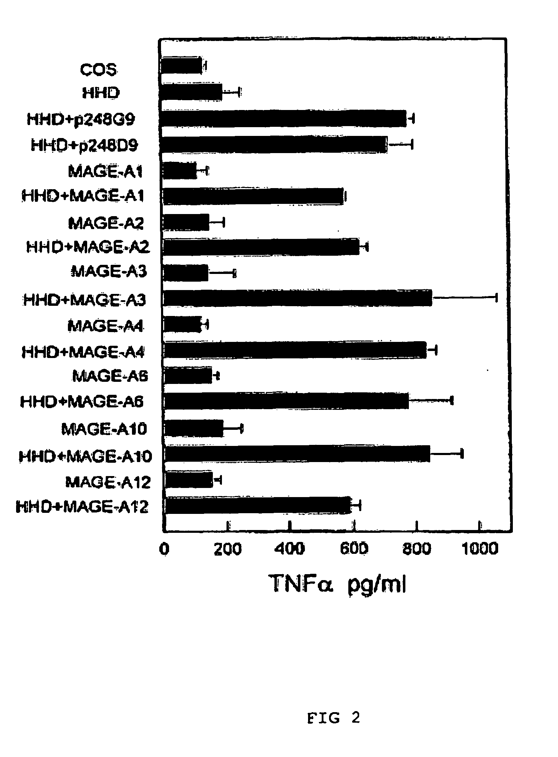

Les résultats sont illustrés par la Figure 2.

Ces résultats montrent que la lignée CTL248 répond à la stimulation par les cellules COS co-exprimant HHD et un antigène MAGE-A. Tous les antigènes MAGE-A1, -A2,A3,-A4,-A6,-A10 et -A12, sont reconnus par cette lignée.

En revanche, on n'observe aucune réponse aux cellules COS transfectées séparément par la construction HHD ou par l'ADNc d'un antigène MAGE-A.

### 2) Stimulation par des cellules tumorales humaines HLA-A*0201 exprimant des antigènes MAGE-A.

Les lignées tumorales HLA-A*0201 suivantes ont été utilisées :
- lignées exprimant au moins un antigène MAGE-A : M44 et M113 (mélanome) ; OBR (cancer de la vessie) ;
- lignées n'exprimant pas d'antigènes MAGE-A : MCF-7 (cancer du sein) et Caco-2 (cancer colon).

Le profil d'expression HLA-A*0201 et MAGE-A de ces lignées est résumé dans le Tableau III ci-dessous.

**Tableau III**

| **Lignée cellulaire** | **HLA-A*0201** | **MAGE-A1** | **MAGE-A2** | **MAGE-A3** | **MAGE-A4** | **MAGE-A6** | **MAGE-A10** | **MAGE-A12** |
|---|---|---|---|---|---|---|---|---|
| **M44** | + | + | + | + | + | - | - | - |
| **M113** | + | - | + | + | - | - | - | - |
| **OBR** | + | - | - | - | - | + | + | - |
| **Caco-2** | + | - | - | - | - | - | - | - |
| **MCF-7** | + | - | - | - | - | - | - | - |

La lignée CTL248 a été stimulée avec chacune des lignées tumorales mentionnées ci-dessus.

La stimulation est évaluée par détection de la sécrétion de TNF-α, comme décrit ci-dessus.

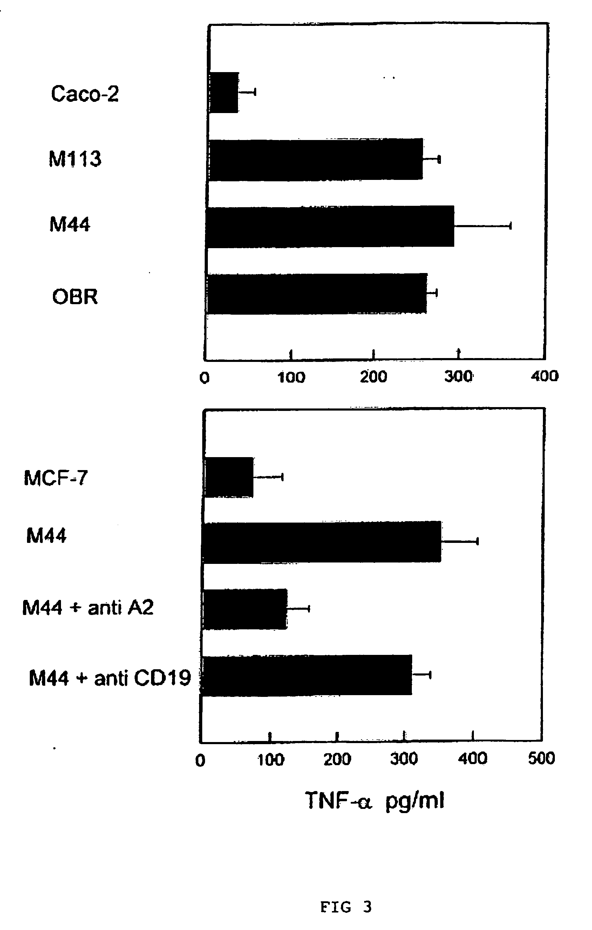

Les résultats sont illustrés par la Figure 3A, qui montre que les cellules CTL248 répondent à la stimulation par les cellules MAGE-A+, quel que soit l'antigène MAGE-A exprimé, mais ne répondent pas aux cellules MCF-7 et Caco-2 qui n'expriment pas d'antigène MAGE-A.

Pour confirmer que la reconnaissance des cellules tumorales est bien restreinte par HLA-A*0201, les cellules CTL248 ont été stimulées par les cellules M44 préincubées pendant une heure avec un anticorps monoclonal anti-HLA-A*0201 (BB7.2) ou avec un anticorps non-pertinent (anti-CD 19), et la production de TNFα a été mesurée comme décrit ci-dessus.

Les résultats illustrés par la Figure 3B, montrent que seul l'anticorps BB7.2, qui bloque HLA-A*0201 inhibe la réponse des cellules CTL248.

Les résultats des expérimentations 1 et 2 ci-dessus montre que les CTL induites par p248V9 reconnaissent un épitope apprêté naturellement, commun aux antigènes MAGE-A1,-A2,-A3,-A4,-A6,-A 10, et -A 12.

### Induction de CTL humains spécifiques par le peptide p248V9.

La capacité de p248V9 à induire des CTL *in vitro* à partir de cellules mononucléées du sang périphérique (PMBC) de donneurs sains a été testée comme suit.
Les PBMC sont obtenues, à partir de prélèvements sanguins par leucocytaphérèse sur des donneurs sains, après centrifugation à 2000 rpm pendant 20 min sur gradient de Ficoll/Hypaque (AMERSHAM). Après 3 lavages en NaCl 0.9%, elles sont resuspendues dans du milieu complet (RPMI 1640 supplémenté avec 10% de sérum humain AB inactivé par la chaleur, 40 µg/mL de gentamicine (PANPHARMA) et 2 µM de L-glutamine (GIBCO)), et incubées à 37°C pendant 2 heures. Après incubation, les cellules non-adhérentes sont prélevées. Les cellules adhérentes sont différenciées en cellules dendritiques par mise en culture dans une poche en TEFLON à raison de 3×10⁶ cellules/mL dans du milieu complet supplémenté avec 500 UI/mL de GM-CSF (R & D SYSTEMS) et 500 UI/mL d'IL-4 (R & D SYSTEMS). Au septième jour, des agents de maturation (100 ng/ml de polyI:C et 2 µg/ml d'anticorps anti-CD40) sont ajoutés à la culture. Après 24 heures, les cellules dendritiques matures sont chargées avec le peptide p248V9 par incubation pendant 2 heures avec 10 µM de peptide en présence de 5 µg/ml de β2-microglobuline, puis irradiées à 3500 rads ; elles sont ensuite lavées pour éliminer le peptide libre.Des cellules CD8+ sont isolées à partir des cellules non-adhérentes à l'aide de microbilles couplées à un anticorps anti-CD8 (MILTENYI BIOTEC).

2×10⁵ cellules CD8+ sont stimulées avec 2×10⁴ cellules dendritiques chargées avec le peptide, dans du milieu complet supplémenté avec 1000 UI/ml d'IL-6 et 5 UI/ml d'IL-12 dans un volume final de 100 µl/puits dans une plaque à 96 puits. A partir du septième jour les cultures sont restimulées *in vitro* chaque semaine, avec les cellules dendritiques chargées par le peptide en présence de 20 UI/ml d'IL-2 et 10 ng/ml d'IL-7. Après la troisième restimulation, les cellules CD8+ sont collectées et stimulées avec des cellules B allogéniques HLA-A*0201 transformées par EBV, dans un rapport 1/2, en présence de 10 µM de peptide pendant 16 heures.

Les cellules CD8+ productrices d'IFN-γ sont purifiées en utilisant un kit du commerce (IFN-γ secretion assay-cell enrichment and détection kit, MILTENYI BIOTECH). Les cellules ainsi purifiées sont cultivées pendant une semaine dans du milieu complet supplémenté avec 20 UI/ml d'IL-2 et 10 ng/ml IL-7.

La réponse de ces cellules CD8+ à des cellules T2 chargées avec l'un des peptides p248V9, p248G9 ou p248D9, ou avec un peptide non-pertinent (HIVgag76), ou à des cellules tumorales HLA-A*0201+MAGE-A+ (M44 et M113) ou HLA-A*0201+MAGE-A (Caco-2) est évalué par dosage de la production d'IFNγ intra-cellulaire.

Les cellules CD8+ sont incubées avec les cellules de la lignée tumorale testée, en présence de 20 µg/ml de BREFELDINE-A (SIGMA). Après 6 heures, elles sont lavées, marquées avec un anticorps anti-CD8 conjugué à la r-phycoérythrine (CALTAG LABORATORIES) dans du PBS pendant 25 min à 4°C, lavées et fixées avec du paraformaldéhyde à 4%. Elles sont ensuites perméabilisées par de la saponine (SIGMA) à 0,2% dans du PBS, et marquées avec un anticorps monoclonal anti-IFNγ conjugué à 1' allophycocyanine (PHARMINGEN).

Les cellules sont ensuite analysées en cytométrie de flux (FACSCalibur^{™} (BECTON DICKINSON) et logiciel CellQuest^{™}).

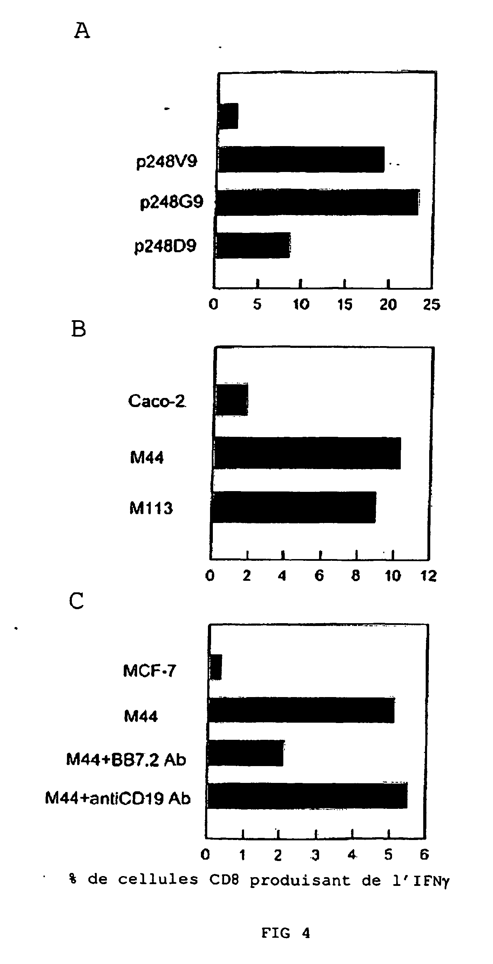

Les résultats sont illustrés par la Figure 4 : (% de cellules productrices d'IFNγ en fonction du peptide ou de la lignée tumorale testée)

La Figure 4A montre une réponse des cellules CD8+ vis-à-vis des cellules T2 chargées avec p248V9, p248G9 ou p248D9, mais pas vis-à-vis des cellules T2 chargées avec le peptide non-pertinent.

La Figure 4B montre une réponse des cellules CD8+ vis-à-vis des lignées tumorales MAGE-A+ M44 et M113 mais pas vis-à-vis de la lignée MAGE-A⁻ Caco-2. La Figure 4C montre en outre que la réponse vis-à-vis de la lignée M44 est fortement inhibée par l'anticorps anti-HLA-A*0201 BB7.2, mais pas par l'anticorps non-pertinent anti-CD 19 Ab.

Ces résultats démontrent que le peptide p248V9 induit des CTL humains capables de reconnaître également les peptides natifs correspondants, et capables de reconnaître des cellules tumorales exprimant des antigènes MAGE-A variés.

### EXEMPLE 3 : IDENTIFICATION D'EPITOPES PRESENTES PAR LA MOLECULE HLA-B*0702 PARTAGES PAR LES ANTIGENES MAGE-A1, -A2, -A3, A4, A6, A10 ET -A12.

L'alignement des séquences des MAGE-A1,-A2,-A3,-A4,-A6,-A10 et -A12 révèle un groupe de 9-mers identiques dans les séquences MAGE-A1,-A2,-A3 et -A6. Le peptide issu de MAGE-A4 qui a une leucine en P2 peut être modifié dans cette position par une substitution L→P. Le peptide issu de MAGE-A12 possède une phénylalanine en P3, mais partage avec les peptides issus de MAGE-A1,-A2,-A3,-A4, et -A6 la séquence TKAEML (positions P4-P9). Seul le peptide issu de MAGE-A10 ne contient pas de séquence d'au moins 5 acides aminés commune avec les peptides issus des autres antigènes MAGE-A.

Les différents groupes de peptides natifs et le peptide variant sont représentés dans le Tableau IV ci-dessous. Les résidus au niveau desquels les peptides natifs diffèrent sont indiqués en caractères gras.

**Tableau IV**

| Antigène | Peptides natifs | Peptide variant |
|---|---|---|
| MAGE-A1 | EPVTKAEML (SEO ID No : 37) | EPVTKAEML (SEQ ID No : 37) |
| MAGE-A2 | EPVTKAEML (SEQ ID No : 37) | |
| MAGE-A3 | EPVTKAEML (SEQ ID No : 37) | |
| MAGE-A4 | ELVTKAEML (SEQ ID No : 39) | |
| MAGE-A6 | EPVTKAEML (SEQ ID No : 37) | |
| MAGE-A10 | EPITKAEIL (SEQ ID No : 40) | |
| MAGE-A12 | EPFTKAEML (SEQ ID No : 41) | |

### EXEMPLE 4 : IDENTIFICATION D'EPITOPES PRESENTES PAR LA MOLECULE HLA-A*0201 PARTAGES PAR LES ANTIGENES HER1, HER2, HER3 ET HER4.

### I : Identification de peptides candidats et construction de variants potentiellement plus immunogènes :

L'alignement des séquences des quatre protéines HER a été effectué comme décrit dans l'exemple 1 et révèle 6 groupes de 9-mers et/ou de 10-mers qui présentent une homologie complète dans la région P3-P8/9. Pour chaque groupe a été défini un variant qui devait avoir une forte affinité pour HLA-A*0201 et être capable de stimuler une réponse cytotoxique spécifique des peptides natifs. Les modifications portent sur les positions d'ancrage P2 et P9/10 (substitution de l'acide aminé d'origine autre que L/V/M/I par une L en P2 et par une V en P9/10) et sur la position P1 (substitution de l'acide aminé par une Y).

Les différents groupes de peptides natifs et les peptides variants sont représentés dans le Tableau V ci-dessous. Les résidus au niveau desquels les peptides natifs diffèrent sont indiqués en caractères gras.

**Tableau V**

| **Antigène** | **Position du groupe de peptides** | **Peptides natifs** | **Peptides variants** |
|---|---|---|---|
| HER2 | 722 | KVKVLGSGA (SEQ ID No : 15) | YVKVLGSGV (SEQ ID No : 19) |
| HER3 | | KLKVLGSGV (SEQ ID No : 16) | |
| HER4 | | RVKVLGSGA (SEQ ID No : 17) | |
| HER1 | | KIKVLGSGA (SEQ ID No : 18) | |
| HER2 | 845 | DLAARNVLV (SEQ ID No : 20) | YLAARNVLV (SEQ ID No : 22) |
| HER3 | | NLAARNVLL (SEQ ID No : 21) | |
| HER4 | | OLAARNVLV (SEQ ID No : 20) | |
| HER1 | | DLAARNVLV (SEQ ID No : 20) | |
| HER2 | 904 | DVWSYGVTV (SEQ ID No : 23) | YVWSYGVTV (SEQ ID No : 25) |
| HER3 | | DVWSYGVTV (SEQ ID No : 23) | |
| HER4 | | DVWSYGVTI (SEQ ID No : 24) | |
| HER1 | | DVWSYGVRV (SEQ ID No : 23) | |
| HER2 | 911 | TVWELMTFGA (SEQ ID No : 26) | YVWELMTFGV (SEQ ID No : 4) |
| HER3 | | TVWELMTFGA (SEQ ID No : 26) | |
| HER4 | | TIWELMTFGG (SEQ ID No : 27) | |
| HER1 | | TVWELMTFGS (SEQ ID No : 28) | |
| HER2 | 933 | DLLEKGERL (SEQ ID No : 29) | YLLEKGERL (SEQ ID No : 31) |
| HER3 | | DLLEKGERL (SEO ID No : 29) | |
| HER4 | | DLLEKGERL (SEQ ID No : 29) | |
| HER1 | | SILEKGERL (SEO ID No : 30) | |
| HER2 | 945 | PICTIDVYMI (SEQ ID No : 32) | YICTIDVYMV (SEQ ID No : 36) |
| HER3 | | QICTIDVYMV (SEQ ID No : 33) | |
| HER4 | | PICTIDVYMV (SEQ ID No : 34) | |
| HER1 | | PICTIDVYKI (SEQ ID No : 35) | |

### II : Affinité pour HLA-A*0201 des peptides sélectionnés :

L'affinité relative (RA) des peptides variants sélectionnés pour HLA-A*0201 a été mesurée comme décrit dans l'exemple 1.

Les résultats sont résumés dans le tableau VI et montrent que seul le variant p911Y1V9 présente une affinité de liaison importante (RA= 2,03), compatible avec un caractère immunogénique.

**Tableau VI**

| Peptide | Séquence | RA |
|---|---|---|
| p722Y1V9 | YVKVLGSGV (SEQ ID No : 19) | >100 |
| p845Y | YLAARNVLV (SEQ ID No : 22) | >100 |
| p904Y | YVWSYGVTV (SEQ ID No : 25) | >100 |
| p911Y1V9 | YVWELMTFGV (SEQ ID No : 4) | 2,03 |
| p933Y | YLLEKGERL (SEQ ID No : 31) | >100 |
| p945Y1V9 | YICTIDVYMV (SEQ ID No : 36) | >100 |

### EXEMPLE 4 : IMMNUNOGENICITE DES PEPTIDES HER VARIANTS :

### I : Induction de CTL spécifiques par vaccination avec les peptides variants

Deux lignées de CTL dénommées 1R5 et 2R5 ont été établies à partir des cellules spléniques de souris HHD immunisées avec le peptide variant p911Y1V9, selon le même protocole que celui décrit dans l'exemple 1.

### Tests de cytotoxicité :

Des essais de cytotoxicité ont été effectués, comme décrit dans l'exemple 2. Pour cela, des cellules cibles RMAS-HHD, chargées avec 10µM des peptides à tester (variant ou peptides natifs), ont été incubées en présence de cellules des deux lignées CTL 1R5 et 2R5.

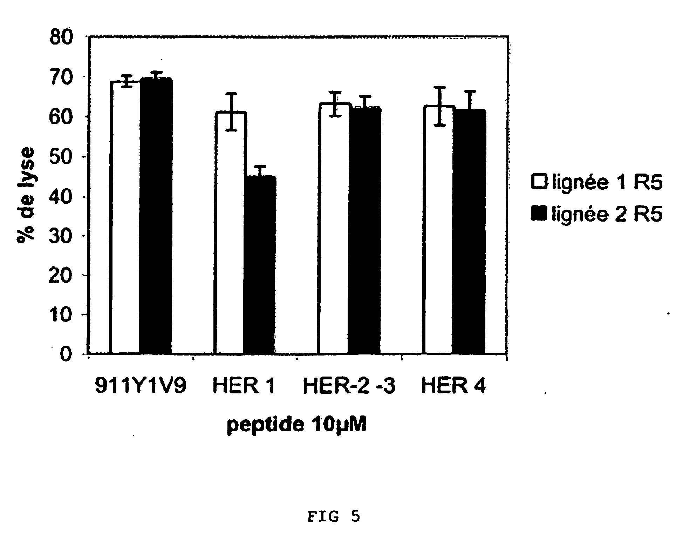

Les pourcentages de lyse spécifique observés en présence des lignées 1R5 (□) et 2R5 (■) et pour les différents peptides testés sont illustrés dans la figure 5.

Les cellules chargées avec le peptide p911Y1V9, ainsi que les peptides natifs HER1 911, HER2,3 911 ou HER4 911, induisent une réponse CTL.

Ces résultats montrent donc que le variant p911Y1V9 génère des CTL qui tuent les cellules cibles RMAS-HHD chargées avec le peptide variant p911Y1V9, mais également avec les peptides natifs HER1 911, HER2,3 911 ou HER4 911.

### Production d'IFNγ intra-cellulaire :

La réponse des cellules de la lignée CTL 1R5 vis-à-vis des cellules RMAS-HHD chargées avec les différents peptides a également été évaluée par le dosage de leur production d'IFNγ intra-cellulaire.

Pour cela, les cellules de la lignée CTL 1R5 sont incubées en présence de cellules RMAS-HHD chargées avec le peptide variant p911Y1V9, les peptides natifs HER1 911, HER2,3 911 ou HER4 911 ou un peptide non pertinent et en présence de 20 µg/ml de BREFELDINE-A (SIGMA). Après 6 heures, les cellules sont lavées, marquées avec un anticorps anti-CD8 conjugué à la r-phycoérythrine et incubées dans du PBS pendant 25 min à 4°C. Elles sont alors de nouveau lavées avant d'être fixées avec une solution de paraformaldéhyde à 4%. Les cellules sont ensuite perméabilisées par la saponine (SIGMA) à 0,2% dans du PBS, et marquées avec un anticorps monoclonal anti-IFNγ conjugué à l'allophococyanine (PHARMINGEN).

Les cellules sont ensuite analysées en cytométrie de flux (FACSCalibur^{™} (BECTON DICKINSON) et logiciel CellQuest^{™}).

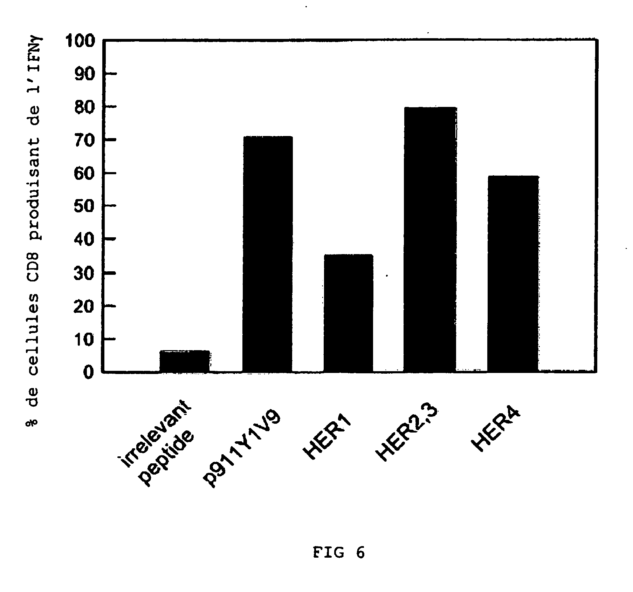

Les résultats sont illustrés dans la figure 6 : (% de cellules productrices d'IFNγ en présence des cellules RMAS-HHD chargées avec les différents peptides à tester).

La figure 6 montre, comme décrit dans la figure 5, une réponse des cellules CD8+ de la lignée CTL1R5 vis-à-vis des cellules RMAS-HHD chargées avec le peptide variant p911Y1V9, avec les peptides natifs HER1 911, HER2,3 911 ou HER4 911, mais pas vis-à-vis des cellules RMAS-HHD chargées avec le peptide non pertinent.

Ces résultats confirment que le peptide p911Y1V9 induit des CTL capables de reconnaître ce peptide variant, mais également les peptides natifs dont il est dérivé.

## Revendications

1. Peptide immunogène comportant un épitope présenté par une molécule HLA de classe I et partagé par au moins deux antigènes d'une même famille multigénique, **caractérisé en ce qu'**il est sélectionné parmi les peptides définis par la séquence YLEYRQVPV (SEQ ID No : 3) et par la séquence YVWELMTFGV (SEQ ID No : 4).

2. Polynucléotide codant pour un peptide selon la revendication 1.

3. Composition comprenant au moins un peptide selon la revendication 1 ou un polynucléotide selon la revendication 2.

4. Composition selon la revendication 3, **caractérisée en ce qu'**il s'agit d'une composition multiépitopique comprenant en outre un ou plusieurs autre(s) peptide(s) immunogène(s) ou un ou plusieurs polynucléotide(s) codant pour le(s)dits peptide(s).

5. Composition selon la revendication 4, **caractérisée en ce qu'**il s'agit d'un polypeptide chimérique comprenant au moins une copie d'un peptide selon la revendication 1, et au moins une copie d'un autre peptide immunogène, ou d'un polynucléotide codant pour ledit polypeptide chimérique.

6. Utilisation d'un peptide selon la revendication 1, d'un polynucléotide selon la revendication 2, ou d'une composition selon une quelconque des revendications 3 à 5, pour l'obtention d'un médicament.

7. Utilisation selon la revendication 6, **caractérisée en ce que** ledit médicament est destiné à l'immunothérapie anti-tumorale.

8. Utilisation selon la revendication 7, **caractérisée en ce que** ledit médicament est destiné à l'immunothérapie de tumeurs exprimant au moins un gène de la famille MAGE-A.

9. Utilisation selon la revendication 7, **caractérisée en ce que** ledit médicament est destiné à l'immunothérapie de tumeurs exprimant au moins un gène de la famille HER.

10. Utilisation selon une quelconque des revendications 6 à 9 , **caractérisée en ce que** ledit médicament est destiné au traitement de patients HLA-A*0201.

## Claims

1. An immunogenic peptide containing an epitope presented by a class I HLA molecule and shared by at least two antigens of one and the same multigenic family, **characterized in that** it is selected from the peptides defined by the sequence YLEYRQVPV (SEQ ID No : 3) and by the sequence YVWELMTFGV (SEQ ID No : 4).

2. A polynucleotide coding for a peptide according to Claim 1.

3. A composition comprising at least one peptide according to Claim 1 or a polynucleotide according to Claim 2.

4. The composition according to Claim 3, **characterized in that** it is a multiepitopic composition also comprising one or more other immunogenic peptide(s) or one or more polynucleotides coding for said peptide(s).

5. The composition according to Claim 4, **characterized in that** it is a chimeric polypeptide comprising at least one copy of a peptide according to Claim 1 and at least one copy of another immunogenic peptide, or a polynucleotide coding for said chimeric polypeptide.

6. Use of a peptide according to Claim 1, a polynucleotide according to Claim 2 or a composition according to any one of Claims 3 to 5, for the preparation of a drug.

7. The use according to Claim 6, **characterized in that** said drug is intended for antitumoural immunotherapy.

8. The use according to Claim 7, **characterized in that** said drug is intended for the immunotherapy of tumours expressing at least one gene of the MAGE-A family.

9. The use according to Claim 7, **characterized in that** said drug is intended for the immunotherapy of tumours expressing at least one gene of the HER family.

10. Use according to any one of Claims 6 to 9, **characterized in that** said drug is intended for the treatment of HLA-A*0201 patients.

## Patentansprüche

1. Immunogenes Peptid mit einem Epitop, das von einem HLA-Molekül der Klasse I dargestellt wird und von mindestens zwei Antigenen ein und derselben Multigenfamilie geteilt wird, **dadurch gekennzeichnet, dass** es aus den Peptiden ausgewählt ist, die durch die Sequenz YLEYRQVPV (SEQ ID Nr.: 3) und durch die Sequenz YVWELMTFGV (SEQ ID Nr.: 4) definiert sind.

2. Codierendes Polynukleotid für ein Peptid nach Anspruch 1.

3. Zusammensetzung mit mindestens einem Peptid nach Anspruch 1 oder einem Polynukleotid nach Anspruch 2.

4. Zusammensetzung nach Anspruch 3, **dadurch gekennzeichnet, dass** es sich um eine Zusammensetzung mit mehreren Epitopen handelt, die außerdem ein oder mehrere andere(s) immunogene(s) Peptid(e) oder ein oder mehrere codierende(s) Polynukleotid(e) für das (die) Peptid(e) umfasst.

5. Zusammensetzung nach Anspruch 4, **dadurch gekennzeichnet, dass** es sich um ein chimäres Polypeptid mit mindestens einer Kopie eines Peptids nach Anspruch 1 und mindestens einer Kopie eines anderen immunogenen Peptids oder eines codierenden Polynukleotids für das chimäre Polypeptid handelt.

6. Verwendung eines Peptids nach Anspruch 1, eines Polynukleotids nach Anspruch 2 oder einer Zusammensetzung nach einem der Ansprüche 3 bis 5 zum Erhalten eines Medikaments.

7. Verwendung nach Anspruch 6, **dadurch gekennzeichnet, dass** das Medikament für die Antitumor-Immuntherapie bestimmt ist.

8. Verwendung nach Anspruch 7, **dadurch gekennzeichnet, dass** das Medikament für die Immuntherapie von Tumoren, die mindestens ein Gen der MAGE-A-Familie exprimieren, bestimmt ist.

9. Verwendung nach Anspruch 7, **dadurch gekennzeichnet, dass** das Medikament für die Immuntherapie von Tumoren, die mindestens ein Gen der HER-Familie exprimieren, bestimmt ist.

10. Verwendung nach einem der Ansprüche 6 bis 9, **dadurch gekennzeichnet, dass** das Medikament für die Behandlung von HLA-A*0201-Patienten bestimmt ist.


### SEQUENCE LISTING

<110> INSERM
   IGR
   GRAFF-DUBOIS, Stéphanie
   KOSMATOPOULOS, Kostas
<120> EPITOPES PEPTIDIQUES COMMUNS A DES ANTIGENES D'UNE MEME FAMILLE MULTIGENIQUE
<130> MJPah1534/1
<160> 41
<170> PatentIn version 3.1
<210> 1
   <211> 9
   <212> PRT
   <213> Homo sapiens
<400> 1
   Ile Val Gly Ala Glu Thr Phe Tyr Val
   1 5
<210> 2
   <211> 10
   <212> PRT
   <213> Homo sapiens
<400> 2
   Arg Pro His Glu Arg Asn Gly Phe Thr Val
   1 5 10
<210> 3
   <211> 9
   <212> PRT
   <213> Artificial sequence
<220>
   <223> peptide modifié
   <400> 3
   Tyr Leu Glu Tyr Arg Gln Val Pro Val
   1 5
<210> 4
   <211> 10
   <212> PRT
   <213> Artificial sequence
<220>
   <223> peptide modifié
   <400> 4
   Tyr Val Trp Glu Leu Met Thr Phe Gly Val
   1 5 10
<210> 5
   <211> 10
   <212> PRT
   <213> Homo sapiens
<400> 5
   Cys Leu Gly Leu Ser Tyr Asp Gly Leu Leu
   1 5 10
<210> 6
   <211> 9
   <212> PRT
   <213> Homo sapiens
<400> 6
   Tyr Leu Glu Tyr Arg Gln Val Pro Gly
   1 5
<210> 7
   <211> 9
   <212> PRT
   <213> Homo sapiens
<400> 7
   Tyr Leu Glu Tyr Arg Gln Val Pro Asp
   1 5
<210> 8
   <211> 9
   <212> PRT
   <213> Homo sapiens
<400> 8
   Tyr Glu Phe Leu Trp Gly Pro Arg Ala
   1 5
<210> 9
   <211> 9
   <212> PRT
   <213> Homo sapiens
<400> 9
   Phe Leu Trp Gly Pro Arg Ala Leu Val
   1 5
<210> 10
   <211> 9
   <212> PRT
   <213> Homo sapiens
<400> 10
   Phe Leu Trp Gly Pro Arg Ala Leu Ile
   1 5
<210> 11
   <211> 9
   <212> PRT
   <213> Homo sapiens
<400> 11
   Phe Leu Trp Gly Pro Arg Ala Leu Ala
   1 5
<210> 12
   <211> 10
   <212> PRT
   <213> Artificial sequence
<220>
   <223> peptide modifié
   <400> 12
   Tyr Leu Gly Leu Ser Tyr Asp Gly Leu Leu
   1 5 10
<210> 13
   <211> 10
   <212> PRT
   <213> Artificial sequence
<220>
   <223> peptide modifié
   <400> 13
   Tyr Leu Gly Leu Ser Tyr Asp Gly Leu Val
   1 5 10
<210> 14
   <211> 9
   <212> PRT
   <213> Artificial sequence
<220>
   <223> peptide modifié
   <400> 14
   Tyr Leu Phe Leu Trp Gly Pro Arg Ala
   1 5
<210> 15
   <211> 9
   <212> PRT
   <213> Homo sapiens
<400> 15
   Lys Val Lys Val Leu Gly Ser Gly Ala
   1 5
<210> 16
   <211> 9
   <212> PRT
   <213> Homo sapiens
<400> 16
   Lys Leu Lys Val Leu Gly Ser Gly Val
   1 5
<210> 17
   <211> 9
   <212> PRT
   <213> Homo sapiens
<400> 17
   Arg Val Lys Val Leu Gly Ser Gly Ala
   1 5
<210> 18
   <211> 9
   <212> PRT
   <213> Homo sapiens
<400> 18
   Lys Ile Lys Val Leu Gly Ser Gly Ala
   1 5
<210> 19
   <211> 9
   <212> PRT
   <213> Artificial sequence
<220>
   <223> peptide modifié
   <400> 19
   Tyr Val Lys Val Leu Gly Ser Gly Val
   1 5
<210> 20
   <211> 9
   <212> PRT
   <213> Homo sapiens
<400> 20
   Asp Leu Ala Ala Arg Asn Val Leu Val
   1 5
<210> 21
   <211> 9
   <212> PRT
   <213> Homo sapiens
<400> 21
   Asn Leu Ala Ala Arg Asn Val Leu Leu
   1 5
<210> 22
   <211> 9
   <212> PRT
   <213> Artificial sequence
<220>
   <223> peptide modifié
   <400> 22
   Tyr Leu Ala Ala Arg Asn Val Leu Val
   1 5
<210> 23
   <211> 9
   <212> PRT
   <213> Homo sapiens
<400> 23
   Asp Val Trp Ser Tyr Gly Val Thr Val
   1 5
<210> 24
   <211> 9
   <212> PRT
   <213> Homo sapiens
<400> 24
   Asp Val Trp Ser Tyr Gly Val Thr Ile
   1 5
<210> 25
   <211> 9
   <212> PRT
   <213> Artificial sequence
<220>
   <223> peptide modifié
   <400> 25
   Tyr Val Trp Ser Tyr Gly Val Thr Val
   1 5
<210> 26
   <211> 10
   <212> PRT
   <213> Homo sapiens
<400> 26
   Thr Val Trp Glu Leu Met Thr Phe Gly Ala
   1 5 10
<210> 27
   <211> 10
   <212> PRT
   <213> Homo sapiens
<400> 27
   Thr Ile Trp Glu Leu Met Thr Phe Gly Gly
   1 5 10
<210> 28
   <211> 10
   <212> PRT
   <213> Homo sapiens
<400> 28
   Thr Val Trp Glu Leu Met Thr Phe Gly Ser
   1 5 10
<210> 29
   <211> 9
   <212> PRT
   <213> Homo sapiens
<400> 29
   Asp Leu Leu Glu Lys Gly Glu Arg Leu
   1 5
<210> 30
   <211> 9
   <212> PRT
   <213> Homo sapiens
   <400> 30
   Ser Ile Leu Glu Lys Gly Glu Arg Leu
   1 5
<210> 31
   <211> 9
   <212> PRT
   <213> Artificial sequence
<220>
   <223> peptide modifié
   <400> 31
   Tyr Leu Leu Glu Lys Gly Glu Arg Leu
   1 5
<210> 32
   <211> 10
   <212> PRT
   <213> Homo sapiens
<400> 32
   Pro Ile Cys Thr Ile Asp Val Tyr Met Ile
   1 5 10
<210> 33
   <211> 10
   <212> PRT
   <213> Homo sapiens
<400> 33
   Gln Ile Cys Thr Ile Asp Val Tyr Met Val
   1 5 10
<210> 34
   <211> 10
   <212> PRT
   <213> Homo sapiens
<400> 34
   Pro Ile Cys Thr Ile Asp Val Tyr Met Val
   1 5 10
<210> 35
   <211> 10
   <212> PRT
   <213> Homo sapiens
<400> 35
   Pro Ile Cys Thr Ile Asp Val Tyr Lys Ile
   1 5 10
<210> 36
   <211> 10
   <212> PRT
   <213> Artificial sequence
<220>
   <223> peptide modifié
   <400> 36
   Tyr Ile Cys Thr Ile Asp Val Tyr Met Val
   1 5 10
<210> 37
   <211> 9
   <212> PRT
   <213> Homo sapiens
   <400> 37
   Glu Pro Val Thr Lys Ala Glu Met Leu
   1 5
<210> 38
   <211> 10
   <212> PRT
   <213> Homo sapiens
<400> 38
   Arg Glu Pro Val Thr Lys Ala Glu Met Leu
   1 5 10
<210> 39
   <211> 9
   <212> PRT
   <213> Homo sapiens
<400> 39
   Glu Leu Val Thr Lys Ala Glu Met Leu
   1 5
<210> 40
   <211> 9
   <212> PRT
   <213> Homo sapiens
<400> 40
   Glu Pro Ile Thr Lys Ala Glu Ile Leu
   1 5
<210> 41
   <211> 9
   <212> PRT
   <213> Homo sapiens
<400> 41
   Glu Pro Phe Thr Lys Ala Glu Met Leu
   1               5